(19) Europäisches Patentamt
European Patent Office
Office européen des brevets

(11) Veröffentlichungsnummer: **0 224 099**
**B1**

(12) # EUROPÄISCHE PATENTSCHRIFT

(45) Veröffentlichungstag der Patentschrift: **05.10.88**

(21) Anmeldenummer: **86115510.9**

(22) Anmeldetag: **08.11.86**

(51) Int. Cl.⁴: **C 07 C 43/178**, C 07 C 41/30, C 08 K 5/06, B 01 D 19/04, B 01 F 17/42

(54) **Perfluoralkylgruppen enthaltende Verbindungen, deren Herstellung und Verwendung als Entschäumungsmittel für wässrige Polymerdispersionen.**

(30) Priorität: **23.11.85 DE 3541515**

(43) Veröffentlichungstag der Anmeldung: **03.06.87 Patentblatt 87/23**

(45) Bekanntmachung des Hinweises auf die Patenterteilung: **05.10.88 Patentblatt 88/40**

(84) Benannte Vertragsstaaten: **DE GB NL**

(56) Entgegenhaltungen:
**EP - A - 0 057 443**
**EP - A - 0 121 918**
**DE - A - 2 336 913**
**DE - A - 3 021 447**
**DE - A - 3 306 593**
**US - A - 3 952 066**
**US - A - 4 079 084**

(73) Patentinhaber: **Th. Goldschmidt AG, Goldschmidtstrasse 100 Postfach 101461, D-4300 Essen 1 (DE)**

(72) Erfinder: **Fock, Jürgen, Dr., Mörsenbroicher Weg 114, D-4000 Düsseldorf 30 (DE)**
Erfinder: **Esselborn, Eberhard, Pilotystrasse 21, D-4300 Essen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft Perfluoralkylgruppen enthaltende Verbindungen, welche im Molekül Oxyalkyleneinheiten aufweisen, die an einen aliphatischen Alkohol mit zwei freien Hydroxylgruppen gebunden sind. Die Erfindung betrifft ferner ein Verfahren zur Herstellung dieser Verbindungen in an sich bekannter (analoger) Weise sowie deren Verwendung als Tenside in der Farb- und Lackindustrie, insbesondere als Entschäumungsmittel für wässerige, gegebenenfalls pigmentierte Polymerdispersionen.

Aliphatische organische Fluorverbindungen haben in hohem Masse technisches Interesse gefunden. Die Verbindungen werden als chemische Laser, funktionelle Flüssigkeiten, Hydro- und Oleophobierungsmittel, Antistatika, Tenside, zur Herstellung von Löschschäumen und für viele andere Zwecke, bei denen es um Grenzflächenphänomene geht, eingesetzt. Die Struktur geeigneter Verbindungen, deren Herstellung und Eigenschaften sind in «Tenside», 13 (1976), 1 ff. und in «Chemiker Zeitung» 99 (1975), 477 ff. und 100 (1976) 3 ff. zusammenfassend beschrieben.

Aus der DE-OS 23 36 913 sind Perfluoralkyljodid-modifizierte Monoalylether bekannt, welche durch Umsetzung eines Perfluoralkyljodids und eines Monoallylethers eines Polyols mit wenigstens drei Hydroxylgruppen erhältlich sind. Beispiel einer solchen Verbindung ist

$CH_2OH$
|
$CHOH$
|
$CH_2OCH_2CHCH_2-R_f$
|
J

($R_f$ = Perfluoralkylgruppe)

Verbindungen dieser Art sollen als oberflächenaktive Mittel und als chemische Zwischenprodukte zur Herstellung von Polyestern, Polyethern oder Polysulfiden geeignet sein.

Ein Anwendungsbereich für grenzflächenaktive Verbindungen ist die Farb- und Lackindustrie. Tenside werden als Verlaufmittel, Dispergiermittel oder als Antischaummittel eingesetzt. Eine besondere Bedeutung haben dabei Mittel zum Verhindern der Bildung von Schaum oder zur Beseitigung von Schaum von wässerigen Beschichtungssystemen aus polymeren, organischen Filmbildnern erlangt.

Bei wässerigen Beschichtungssystemen aus polymeren, organischen Filmbildnern kann man zwei Arten von Schaum beobachten. Makroschaum entsteht beim Einrühren von Luft. Die Gasblasen sind von dünnen Lamellen umgeben und grenzen unter Bildung von Polyedern aneinander. Die Lamellen sind durch eingelagerte, oberflächenaktive Verbindungen stabilisiert und können durch Zusatz geeigneter oberflächenaktiver Verbindungen destabilisiert werden. Mikroschaum zeigt sich in Form kleiner kugelförmiger Gasbläschen, die voneinander durch relativ dicke Flüssigkeitsschichten getrennt und in der Lösung oder der geschlossenen Phase der Dispersion verteilt sind. Mikroschaum führt im Anstrichfilm zu sogenannten Nadelstichen (pin-holes). Mittel zum Beseitigen des Mikroschaumes werden allgemein als Entlüftungsmittel bezeichnet. Sie beschleunigen das Aufsteigen der Blasen und erzwingen deren Platzen an der Oberfläche. Als Wirkungsmechanismus wird in der Literatur eine Verringerung der Oberflächenviskosität diskutiert.

Als Entschäumungsmittel für Makroschaum werden Mineralöle, pflanzliche und tierische Öle und Siliconöle sowie modifizierte Silicone, wie Polyoxyalkylen-Polysiloxan-Blockmischpolymerisate, und fluororganische Verbindungen verwendet. Derartige Mittel sind u.a. in den DE-PSen 23 45 335, 24 43 853 und 32 01 479 beschrieben. Die Wirksamkeit der Entschäumungsmittel kann häufig durch Zusatz von ganz oder teilweise hydrophobierter feinteiliger Kieselsäure verbessert werden.

Mittel zum Entlüften von wässerigen Polymerdispersionen sind in der DE-PS 32 18 676 beschrieben. Danach kann man den in solchen Systemen enthaltenen Mikroschaum dadurch beseitigen, dass man ein Mittel zusetzt, das 5 bis 60 Gew.-% eines linearen Polymerisates in organischen Lösungsmitteln gelöst enthält, wobei das Polymerisat mindestens fünf seitenständig gebundene Gruppen aufweist, welche jeweils ein Siliciumatom enthalten, das über eine zweiwertige Kohlenwasserstoffgruppe mit dem Polymerisat verknüpft ist, und wobei das Siliciumatom mindestens eine Gruppe $R^1$ der Formel $O-[C_nH_{2n}O-]_xQ$ trägt, wobei n=2 bis 8, x=1 bis 10, Q ein Alkylrest mit 1 bis 8 Kohlenstoffatomen, ein Arylrest, ein Alkarylrest oder ein Acylrest mit 2 bis 18 Kohlenstoffatomen ist und die Summe der Kohlenstoff- und Sauerstoffatome der Gruppe $R^1 \geq 5$ ist.

Der vorliegenden Erfindung liegt die Aufgabe zugrunde, besonders wirksame Entschäumungsmittel für wässerige, gegebenenfalls pigmentierte Polymerdispersionen zu finden, die insbesondere die Bildung des Makroschaumes verhindern oder bereits gebildeten Schaum zusammenfallen lassen. Dabei soll etwaiger Mikroschaum möglichst gering gehalten oder unterdrückt werden. Darüber hinaus sollen die Verbindungen in möglichst kleinen Konzentrationen wirksam sein.

Es wurde überraschend gefunden, dass Perfluoralkylgruppen aufweisende Polyoxylalkylenether von aliphatischen Alkoholen mit zwei freien Hydroxylgruppen am Startalkohol bestimmter Struktur diese Aufgaben erfüllen.

Ein Gegenstand vorliegender Erfindung sind deshalb Verbindungen der allgemeinen Formel

$R^2$
|
$HO-R^1-OH$
|
$CH_2O(C_nH_{2n}O)_xCH_2CR^3{=}CH-C_mF_{2m+1}$

wobei

$R^1$ eine Kohlenwasserstoffgruppe, welche die beiden Hydroxylgruppen über maximal 3 Kohlenstoffatome miteinander verbindet,

$R^2$ ein Wasserstoffrest, eine Alkyl- oder Arylgruppe oder die Gruppe

$$-CH_2O(C_nH_{2n}O)_xCH_2CR^3=CH-C_mF_{2m+1},$$

$R^3$ ein Wasserstoffrest oder eine Methylgruppe,
n eine Zahl von 2 bis 14, deren Wert innerhalb des Moleküls unterschiedlich sein kann,
m eine Zahl von 2 bis 20,
x eine Zahl $\geq 2$
ist.

Die Gruppe $R^1$ ist vorzugsweise eine Alkylengruppe, insbesondere die Gruppe $-C-CH_2-$ oder $-CH_2-C-CH_2-$.

Die Gruppe $R^2$ ist vorzugsweise eine Alkylgruppe, insbesondere eine Ethylgruppe. Als Arylgruppe ist die Phenylgruppe bevorzugt.

Innerhalb der Gruppen $-C_nH_{2n}O-$ sind die Gruppen mit n=2 und n=3, die durch Anlagerung von Ethylenoxid bzw. Propylenoxid erhalten werden, bevorzugt. Jedoch können auch längerkettige Oxyalkyleneinheiten mit bis zu 14 Kohlenstoffatomen enthalten sein. Durch Auswahl der Alkylenoxide kann die Hydrophilie und damit die Löslichkeit in der Dispersion bzw. die Verträglichkeit mit dem organischen Filmbildner in an sich bekannter Weise beeinflusst werden. Dabei können Oxyalkyleneinheiten unterschiedlicher Kettenlänge im gleichen Molekül blockweise oder alternierend vorliegen.

Die Anzahl der Oxylalkyleneinheiten muss mindestens 2 betragen. Vorzugsweise sind jedoch mehr als 2, insbesondere 5 bis 50, besonders bevorzugt 5 bis 25 Oxyalkyleneinheiten vorhanden. Dabei überwiegt vorzugsweise die Anzahl der Oxyethyleneinheiten.

m gibt die Kettenlänge der perfluorierten Alkylgruppen an. m ist vorzugsweise 4 bis 8. Die Kettenlänge der Perfluoralkylgruppen ist eher durch die Verfügbarkeit der im Handel erhältlichen Perfluorverbindungen als durch Eigenschaftsänderungen begrenzt.

Beispiele von erfindungsgemässen Verbindungen können folgende Struktur haben:

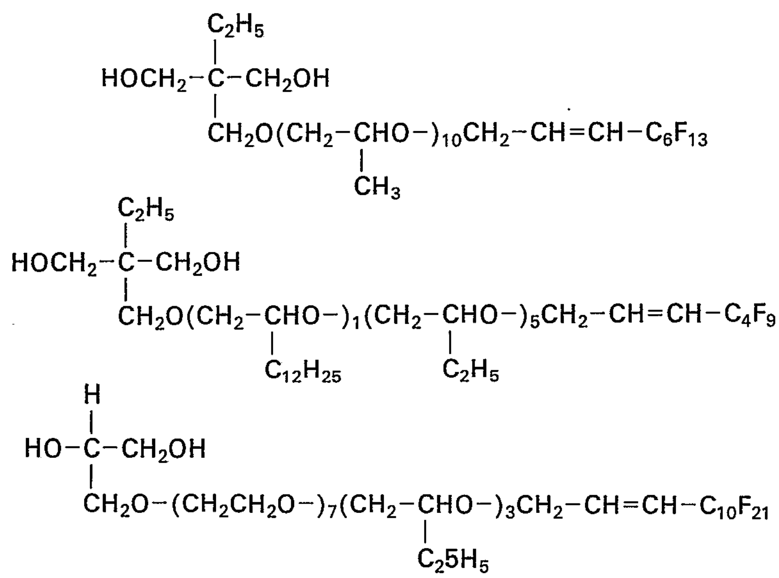

Ein weiterer Gegenstand der Erfindung besteht in dem Verfahren zur Herstellung der erfindungsgemässen Verbindungen. Dies ist dadurch gekennzeichnet, dass man an Verbindungen der allgemeinen Formel

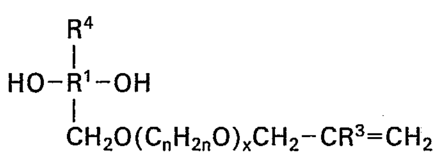

wobei
$R^1$, $R^3$, n und x die angegebene Bedeutung haben,
$R^4$ ein Wasserstoffrest, eine Alkyl- oder Arylgruppe oder die Gruppe

$$-CH_2O(C_nH_{2n}O)_xCH_2-CR^3=CH_2$$

ist,
in an sich bekannter Weise in einem Lösungsmittel und in Gegenwart eines Radikalbildners mit einem Perfluoralkyljodid der allgemeinen Formel $C_mF_{2m+1}J$ umsetzt, das erhaltene Zwischenprodukt in an sich bekannter Weise mit in bezug auf J-Reste mindestens äquimolaren Mengen Alkali- oder Erdalkalihydroxid umsetzt, das gebildete Alkali- oder Erdalkalijodid abtrennt und das Lösungsmittel entfernt.

Der Rest $R^4$ entspricht dem Rest $R^3$ auch mit seinen bevorzugten Bedeutungen mit der Ausnahme, dass die Endgruppe der Polyoxyalkylenkette die Gruppe $CH_2-CR^3=CH_2$ ist.

Die Ausgangsverbindung

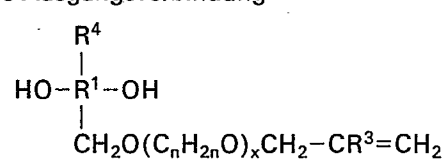

I

ist in an sich bekannter Weise durch Umsetzung der Alkalisalze von gemäss DE-PS 30 23 059 erhältlichen Verbindungen der Formel

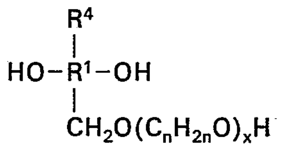

mit Allyl- oder Methallylchlorid entsprechend der DE-OS 30 25 807 zugänglich.

Die Umsetzung der Verbindung der Formel I mit einem Perfluorjodid der Formel $C_mF_{2m+1}J$ zu Verbindungen der Formel

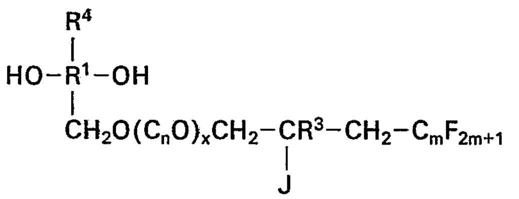 II

erfolgt in an sich bekannter Weise, z.B. entsprechend der DE-OS 23 36 913 in Gegenwart von Radikalbildnern in Gegenwart eines Lösungsmittels. Als Radikalbildner ist Azodiisobutyronitril besonders geeignet. Brauchbar sind ferner Benzoylperoxid oder ein Dialkylperoxid, wie Di-tert.-butylperoxid. Geeignete Lösungsmittel sind Wasser, Aceton, Hexan oder Heptan. Die Reaktion verläuft je nach Art des Radikalbildners bei etwa 50 bis 130° C. Die Reaktionszeit beträgt 1 bis 3 Stunden.

Das so erhaltene Zwischenprodukt der Formel II wird nun in ebenfalls bekannter Weise mit in bezug auf J-Reste mindestens äquimolaren Mengen Alkali- oder Erdalkalihydroxid umgesetzt. Bevorzugt verwendet man NaOH, das man in Form einer wässerigen Lösung einsetzt. Unter Abspaltung des entsprechenden Metalljodides werden die erfindungsgemässen Verbindungen erhalten.

Insbesondere bei Ausgangsverbindungen mit Oxyethyleneinheiten bereitet es mitunter Schwierigkeiten, die Abspaltung des Jods quantitativ durchzuführen. Es hat sich jedoch gezeigt, dass in den meisten Fällen der erfindungsgemässen Verwendung die Anwesenheit einiger Prozente des Zwischenproduktes der allgemeinen Formel II nicht dem gewünschten Effekt abträglich ist.

Das abgespaltene Metalljodid wird zweckmässig als wässerige Lösung abgezogen oder durch Destillation des Wassers und anschliessende Filtration abgetrennt und das Lösungsmittel abdestilliert.

Die erfindungsgemässen Produkte weisen die geforderten Eigenschaften auf. Sie sind in hohem Masse grenzflächenaktiv und erniedrigen die Oberflächenspannung von Wasser und wässerigen Systemen erheblich. Sie sind insbesondere hochwirksame Entschäumungsmittel für wässerige Polymerdispersionen, so dass ihre Verwendung hierfür ein weiterer Gegenstand der Erfindung ist. Die Verbindungen sind darüber hinaus ausgezeichnete Egalisiermittel für wässerige Wachsdispersionen, d.h. sie ermöglichen den gleichmässigen Auftrag und die leichte Verteilung von derartigen Dispersionen auf Oberflächen, wie Wachspolituren auf Möbeln. Sie sind auch als Entschäumer in der Erdölförderung und -verarbeitung einsetzbar. Die Verbindungen sind aufgrund ihrer beiden Hydroxylgruppen als Polyolkomponente zur Herstellung von Polyestern, Polyethern, Polysulfiden, Polyurethanen oder zur Modifizierung von Polysiloxanen geeignet.

Die folgenden Beispiele dienen der weiteren Erläuterung der Erfindung.

*Beispiel 1:*

475 g (ca. 1 Mol) des Polyethers der Formel

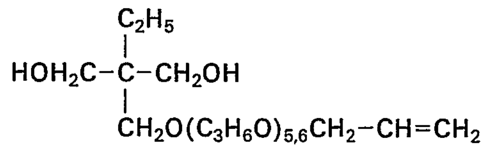

mit einer OH-Zahl von 221 und einer Jodzahl von 53,4 werden zusammen mit der Lösung von 56 g $Na_2S_2O_5$ in 190 g Wasser in einem 1-l-Dreihalskolben vorgelegt und dazu langsam 669 g (ca. 1,5 Mol) Perfluorhexyljodid und 34,7 g Azodiisobuttersäurenitril gegeben. Es wird nun für 2 h auf 80° C und danach für 2 h auf 90° C erhitzt. Danach werden bei 60° C 600 g einer 20%igen wässerigen Natriumhydroxidlösung zugegeben.

Nach Beendigung der Reaktion wird das unumgesetzte Perfluoralkyljodid zusammen mit dem Wasser abdestilliert, wobei sich allmählich als Bodensatz eine konzentrierte wässerige Lösung von Natriumhydroxid und Natriumjodid bildet, die schliesslich abgesaugt wird. Durch Waschen mit 600 ml Wasser und Neutralisation mit verdünnter Salzsäure werden Reste der Lauge und der Salze entfernt. Die wässerige Lösung bildet sich jetzt als obere Phase aus, die ebenfalls abgeschieden wird. Nach Entfernung des restlichen Wassers im Vakuum und anschliessender Filtration erhält man ein klares, leicht gelbliches Produkt.

Das erhaltene Polyether-1.3-diol mit der Perfluorhexylendgruppe hat eine Hydroxylzahl von 124, der Gehalt an Fluor beträgt 30 Gew.-% und an Jod 0,47 Gew.-%.

*Beispiele 2 bis 8:*

Analog Beispiel 1 werden Verbindungen der Formel

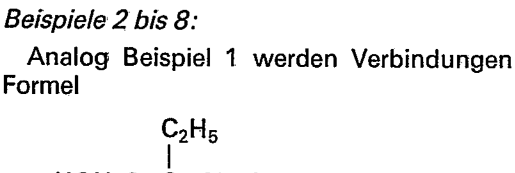

mit unterschiedlichem Oxypropylengehalt mit Verbindungen der Formel $C_mF_{2m+1}J$ umgesetzt. In der Tabelle 1 werden die aus der Hydroxyl- und Jodzahlbestimmung ermittelten Molekulargewichte ($MG_{OHZ}$ bzw. $MG_{JZ}$) der noch nicht mit Perfluoralkyljodid umgesetzten Polyether-1.3-diole, die Werte für x und m sowie die prozentualen Fluor- und Jodgehalte der erhaltenen Produkte angegeben.

*Tabelle 1*

| Beispiel Nr. | $MG_{OHZ}$ | $MG_{JZ}$ | x | m | Fluorgehalt (Gew.-%) | Jodgehalt (Gew.-%) |
|---|---|---|---|---|---|---|
| 2 | 334 | 344 | 2,9 | 6 | 37,5 | 1,7 |
| 3 | 550 | 542 | 6,5 | 6 | 28,4 | 0,9 |
| 4 | 644 | 627 | 8,1 | 6 | 23,5 | 0,6 |
| 5 | 950 | 884 | 13,4 | 6 | 19,9 | 0,8 |
| 6 | 1810 | 1800 | 28,2 | 6 | 11,4 | 2,0 |
| 7 | 644 | 637 | 8,1 | 4 | 19,7 | 1,2 |
| 8 | 644 | 637 | 8,1 | 8 | 30,2 | 1,3 |
| 9 | 950 | 884 | 13,4 | 12 | 31,0 | 1,9 |
| 10 | 1810 | 1800 | 28,2 | 20 | 27,2 | 1,7 |

*Beispiel 11:*

Analog Beispiel 1 wird eine Verbindung der Formel

$$C_2H_5$$
$$|$$
$$HOH_2C-C-CH_2OH$$
$$|$$
$$CH_2O(C_4H_8O)_4(C_{14}H_{28}O)_1CH_2-CH=CH_2$$

(Molgew.$_{OHZ}$ 672)
(Jodzahl 37,5)

mit Perfluorhexyljodid umgesetzt und mit wässeriger KOH in das Endprodukt übergeführt. Die OH-Zahl beträgt 56. Der Fluorgehalt beträgt 24,8 Gew.-%, der Jodgehalt 0,8 Gew.-%.

*Beispiel 12:*

Analog Beispiel 1 wird eine Verbindung der Formel

$$H$$
$$|$$
$$HO-C-CH_2OH$$
$$|$$
$$CH_2O(C_3H_6O)_{5,6}CH_2-CH=CH_2$$

mit Perfluorhexyljodid umgesetzt.

Die OH-Zahl des Endproduktes beträgt 134, der Jodgehalt beträgt 0,6, der Fluorgehalt 31,6 Gew.-%.

*Beispiele 13 bis 18:*

Analog Beispiel 1 werden Verbindungen der Formel

$$C_2H_5$$
$$|$$
$$HOH_2C-C-CH_2OH$$
$$|$$
$$CH_2O(C_2H_4O)_xCH_2-CH=CH_2$$

mit unterschiedlichem Ethylenoxidgehalt mit Verbindungen der Formel $C_mF_{2m+1}J$ umgesetzt. In der Tabelle 2 werden die aus der Hydroxyl- und Jodzahlbestimmung ermittelten Molekulargewichte $MG_{OHZ}$ und $MG_{JZ}$ der noch nicht umgesetzten Polyether-1.3-diole, die Werte für x und m sowie die prozentualen Fluorgehalte der erhaltenen Endprodukte angegeben.

*Tabelle 2*

| Beispiel Nr. | $MG_{OHZ}$ | $MG_{JZ}$ | x | m | Fluorgehalt (Gew.-%) |
|---|---|---|---|---|---|
| 13 | 303 | 310 | 2,9 | 6 | 32,6 |
| 14 | 487 | 507 | 7,1 | 6 | 23 |
| 15 | 908 | 790 | 16,7 | 6 | 20 |
| 16 | 2150 | 2169 | 44,9 | 6 | 7,4 |
| 17 | 487 | 507 | 7,1 | 4 | 20,0 |
| 18 | 487 | 507 | 7,1 | 8 | 33,1 |

*Beispiel 19:*

Analog Beispiel 1 wird eine Verbindung der Formel

$$C_2H_5$$
$$|$$
$$HOH_2C-C-CH_2OH$$
$$|$$
$$CH_2O(C_2H_4O)_8(C_3H_6O)_2CH_2-CH_2-CH=CH_2$$

mit Perfluorhexyljodid umgesetzt. Das Endprodukt hat einen Fluorgehalt von 25,7 Gew.-%.

*Test zur Prüfung der Entschäumungsfähigkeit*

In einem Becherglas mit einem Durchmesser von 6 cm werden 100 g einer 45%igen wässerigen Dispersion eines Copolymerisates auf Basis von Styrol und einem Acrylsäureester (im Handel unter der Bezeichnung Acronal® 170 D erhältlich) in Gegenwart einer erfindungsgemässen Verbindung mit einem Turbinenrührer mit einem Durchmesser von 4 cm 1 Minute lang mit einer Drehzahl von 2500 Upm entsprechend einer Umfangsgeschwindigkeit von 5,2 m/sec gerührt.

Die derart behandelte Dispersion wird unmittelbar nach Abstellen des Rührers in einen 50-ml-Messkolben bis zur Eichmarke gefüllt und danach gewogen. Die Dichte der Kolbenfüllung hängt von der eingerührten Luftmenge ab und stellt ein Mass für die Wirksamkeit des Entschäumers dar.

Die Tabelle 3 zeigt die Menge der erfindungsgemässen Verbindungen oder der nicht erfindungsgemässen Vergleichsverbindungen in Gew.-%, bezogen auf die eingesetzte, nicht gerührte Dispersion und das Volumen der eingerührten Luft pro 100 $cm^3$ gerührter Dispersion unmittelbar nach dem Rühren. Das Volumen der eingerührten Luft pro 100 $cm^3$ gerührter Dispersion beträgt 55,4 $cm^3$, sofern kein Entschäumer eingesetzt wird.

*Tabelle 3*

| Substanz von Beispiel | Luftvolumen ($cm^3$) bei einer Konzentration (Gew.-%) von | | |
|---|---|---|---|
| | 0,2 | 0,1 | 0,02 |
| 1 | 3,6 | 7,0 | 11,4 |
| 2 | 2,3 | 6,6 | 9,0 |
| 3 | 3,2 | 6,8 | 12,2 |
| 4 | 3,8 | 7,8 | 15,8 |
| 5 | 3,6 | 8,8 | 13,4 |
| 6 | 9,4 | 9,6 | 14,2 |
| 7 | 5,6 | 8,2 | 18,0 |
| 8 | 2,0 | 4,4 | 7,6 |
| 9 | 2,3 | 4,7 | 7,5 |
| 10 | 2,7 | 5,5 | 8,9 |
| 11 | 3,5 | 5,9 | 9,0 |
| 12 | 3,6 | 7,0 | 12,2 |
| Vergleichsprodukt | | | |
| A | 5,8 | 9,7 | 18,8 |
| B | 21,4 | — | — |
| C | 18,4 | — | — |
| D | 18,4 | — | — |
| E | 16,5 | — | — |

Vergleichsprodukt A ist ein Gemisch eines Polyoxypropylen-Polysiloxan-Blockmischpolymerisates und hochdisperser Kieselsäure gemäss DE-PS 24 43 853.

Vergleichsprodukt B ist ein Gemisch aus Mineralöl, Silicon und Emulgator, im Handel als Nopco 8034 E erhältlich.

Vergleichsprodukt C ist ein Gemisch aus Fettsäureestern, Paraffin und Silicon, im Handel als Bevaloid I 142 erhältlich.

Vergleichsprodukt D ist ein Gemisch aus Mineralöl, Fettsäureestern und Metallseifen, im Handel als Byk 073 erhältlich.

Vergleichsprodukt E ist ein Gemisch aus Mineralöl, Alkoholen, Emulgatoren und Metallseifen, im Handel als Dehydran A erhältlich.

*Bestimmung der Oberflächenspannungserniedrigung*

Die Bestimmung der Oberflächenspannung von erfindungsgemässe bzw. Vergleichssubstanzen enthaltendem Wasser erfolgt nach DIN 53 914. Hierzu wird zunächst eine 1 gew.-%ige Lösung der zu prüfenden Substanz in bidestilliertem Wasser hergestellt. Als Probegefäss dient ein thermostatisierbares Glasgefäss mit einem Durchmesser von 10 cm. Es wird nun die Kraft K gemessen, die notwendig ist, um einen horizontal zur Flüssigkeitsoberfläche aufgehängten Ring mit dem Radius R aus der Flüssigkeitsoberfläche herauszuziehen:

$$\delta = \frac{K}{4 \pi R}$$

Die in Tabelle 4 wiedergegebenen Werte zeigen die Oberflächenspannung erfindungsgemässer Verbindungen sowie als nicht erfindungsgemässe Vergleichssubstanzen Verbindungen der Formel

$$-C_mF_{2m+1}-(CH_2)_3O-(C_2H_4O)_x-H \qquad III$$

*Tabelle 4*

| Substanz von Beispiel | | Oberflächenspannung δ ($mNm^{-1}$) bei einer Konzentration (Gew.-%) von 1 | 0,1 | 0,01 |
|---|---|---|---|---|
| 13 | | — | 20 | 22 |
| 14 | | 19 | 21 | 24 |
| 15 | | 21 | 23 | 29 |
| 16 | | 24 | 26 | 33 |
| 17 | | 21 | 23 | 27 |
| 18 | | 18 | 20 | 23 |
| Mittelwert | | 20,6 | 22,7 | 27,2 |
| Vergleichssubstanz der Formel III | | | | |
| m | x | | | |
| 6 | 5 | — | 26 | 32 |
| 6 | 13 | 23 | 25 | 32 |
| 6 | 21 | 27 | 28 | 38 |
| 6 | 45 | 27 | 32 | 45 |
| 7 | 4 | — | 28 | 32 |
| 7 | 14 | 24 | 25 | 30 |
| 8 | 8 | 23 | 26 | 34 |
| Mittelwert | | 24,8 | 27,1 | 34,7 |

*Bestimmung der Oberflächenspannung in einer Wachszubereitung*

Erfindungsgemässe Verbindungen werden als Egalisierungsmittel in einer Wachsemulsion eingesetzt und in ihrer Wirkung mit Verbindungen der allgemeinen Formel III verglichen.

Je niedriger die Oberflächenspannung ist, um so besser ist das Egalisiervermögen der Additive, und damit tritt nach der Trocknung eine um so geringere Hof- und Randbildung auf.

Zur Herstellung einer Wachsemulsion werden 5 Gew.-Teile eines emulgatorfreien Montanwachses mit einem Tropfpunkt von 83 bis 89° C, einer Säurezahl von 85 bis 95, einer Verseifungszahl von 87 bis 104, 1 Gew.-Teil Diethanolamin und 27 Gew.-Teile Wasser verwendet.

In Tabelle 5 werden die Oberflächenspannungswerte bei Zusatz von 0,05 Gew.-% der zu prüfenden Verbindungen zur Wachsemulsion wiedergegeben.

*Tabelle 5*

| Substanz von Beispiel | | Oberflächenspannung δ ($mNm^{-1}$) bei Zusatz von 0,05% Substanz |
|---|---|---|
| 13 | | 32 |
| 14 | | 31 |
| 15 | | 33 |
| 16 | | 34 |
| 17 | | 33 |
| 18 | | 31 |
| Mittelwert | | 32 |
| Vergleichssubstanz der Formel III | | |
| m | x | |
| 6 | 5 | 35 |
| 6 | 13 | 36 |
| 6 | 21 | 37 |
| 6 | 45 | 39 |
| 7 | 4 | 34 |
| 7 | 14 | 37 |
| 8 | 8 | 33 |
| Mittelwert | | 36 |

## Patentansprüche

1. Verbindungen der allgemeinen Formel

$$\begin{array}{l} \quad R^2 \\ \quad | \\ HO-R^1-OH \\ \quad | \\ \quad CH_2O(C_nH_{2n}O)_xCH_2CR^3=CH-C_mF_{2m+1} \end{array}$$

wobei

$R^1$ eine Kohlenwasserstoffgruppe, welche die beiden Hydroxylgruppen über maximal 3 Kohlenstoffatome miteinander verbindet,

$R^2$ ein Wasserstoffrest, eine Alkyl- oder Arylgruppe oder die Gruppe

$$-CH_2O(C_nH_{2n}O)_xCH_2CR^3=CH-C_mF_{2m+1},$$

$R^3$ ein Wasserstoffrest oder eine Methylgruppe,

n eine Zahl von 2 bis 14, deren Wert innerhalb des Moleküls unterschiedlich sein kann,

m eine Zahl von 2 bis 20,

x eine Zahl $\geqq 2$

ist.

2. Verfahren zur Herstellung der Verbindungen des Anspruchs 1, dadurch gekennzeichnet, dass man an Verbindungen der allgemeinen Formel

$$\begin{array}{l} \quad R^4 \\ \quad | \\ HO-R^1-OH \\ \quad | \\ \quad CH_2O(C_nH_{2n}O)_xCH_2-CR^3=CH_2 \end{array}$$

wobei

$R^1$, $R^3$, n und x die angegebene Bedeutung haben,

$R^4$ ein Wasserstoffrest, eine Alkyl- oder Arylgruppe oder die Gruppe

$$-CH_2O(C_nH_{2n}O)_xCH_2-CR^3=CH_2$$

ist,

in an sich bekannter Weise in einem Lösungsmittel und in Gegenwart eines Radikalbildners mit einem Perfluoralkyljodid der allgemeinen Formel $C_mF_{2m+1}J$ umsetzt, das erhaltene Zwischenprodukt in an sich bekannter Weise mit in bezug auf J-Reste mindestens äquimolaren Mengen Alkali- oder Erdalkalihydroxid umsetzt, das gebildete Alkali- oder Erdalkalijodid abtrennt und das Lösungsmittel entfernt.

3. Verwendung der Verbindungen des Anspruchs 1 als Tenside in der Farb- und Lackindustrie, insbesondere als Entschäumungsmittel für wässerige, gegebenenfalls pigmentierte Polymerdispersionen.

## Claims

1. Compounds of the general formula

$$\begin{array}{l} \quad R^2 \\ \quad | \\ HO-R^1-OH \\ \quad | \\ \quad CH_2O(C_nH_{2n}O)_xCH_2CR^3=CH-C_mF_{2m+1} \end{array}$$

where

$R^1$ is a hydrocarbon group which connects the two hydroxyl groups to one another via a maximum of 3 carbon atoms,

$R^2$ is a hydrogen radical, an alkyl or aryl group, or the

$$-CH_2O(C_nH_{2n}O)_xCH_2CR^3=CH-C_mF_{2m+1}$$

group,

$R^3$ is a hydrogen radical or a methyl group,

n is a number between 2 and 14 whose value may be different within the molecule,

m is a number of between 2 and 20,

x is a number $\geqq 2$.

2. Process for the preparation of the compounds of Claim 1, characterized in that compounds of the general formula

$$\begin{array}{l} \quad R^4 \\ \quad | \\ HO-R^1-OH \\ \quad | \\ \quad CH_2O(C_nH_{2n}O)_xCH_2-CR^3=CH_2 \end{array}$$

where

$R^1$, $R^3$, n and x have the specified meaning, and

$R^4$ is a hydrogen radical, an alkyl or aryl group, or the $-CH_2O(C_nH_{2n}O)_xCH_2-CR_3=CH_2$ group, are reacted in a manner known per se with a perfluoroalkyliodide of the general formula $C_mF_{2m+1}I$ in a solvent and in the presence of a free-radical generator, the intermediate obtained is reacted in a manner known per se with at least equimolar amounts, relative to the I radicals, of an alkali metal hydroxide or alkaline-earth metal hydroxide, the alkali metal iodide or alkaline-earth metal iodide formed is separated off, and the solvent is removed.

3. Use of the compounds of Claim 1 as surfactants in the paints industry, in particular as antifoaming agents for aqueous, optionally pigmented polymer dispersions.

## Revendications

1. Composés de formule générale

$$\begin{array}{l} \quad R^2 \\ \quad | \\ HO-R^1-OH \\ \quad | \\ \quad CH_2O(C_nH_{2n}O)_xCH_2CR^3=CH-C_mF_{2m+1} \end{array}$$

dans laquelle

$R^1$ est un groupe hydrocarboné, qui relie l'un à l'autre les deux groupes hydroxyle par l'intermédiaire d'au plus 3 atomes de carbone,

$R^2$ est un radical hydrogène, un groupe alkyle ou aryle, ou encore le groupe

$$-CH_2O(C_nH_{2n}O)_xCH_2CR^3=CH-C_mF_{2m+1},$$

$R^3$ est un radical hydrogène ou un groupe méthyle,

n est un nombre de 2 à 14, dont la valeur peut varier à l'intérieur de la molécule,

m est un nombre de 2 à 20,
x est un nombre ≧ 2.

2. Procédé pour la préparation des composés selon la revendication 1, caractérisé en ce qu'on fait réagir des composés de formule générale

$$\begin{array}{l} \quad\;\; R^4 \\ \quad\;\; | \\ HO-R^1-OH \\ \quad\;\; | \\ \quad\;\; CH_2O(C_nH_{2n}O)_xCH_2-CR^3=CH_2 \end{array}$$

dans laquelle
$R^1$, $R^3$, n et x ont les significations données ci-dessus,
$R^4$ est un radical hydrogène, un groupe alkyle ou aryle, ou le groupe

$$-CH_2O(C_nH_{2n}O)_xCH_2-CR^3=CH_2,$$

d'une manière connue en soi, dans un solvant et en présence d'un initiateur radicalaire, avec une iodure de perfluoralkyle de formule générale $C_mF_{2m+1}I$, qu'on fait réagir le produit intermédiaire obtenu, d'une manière connue en soi, sur des quantités au moins équimolaires, par rapport aux radicaux I, d'hydroxyde de métaux alcalins ou de métaux alcalino-terreux, qu'on sépare l'iodure de métal alcalin ou de métal alcalino-terreux ainsi formé, et qu'on chasse le solvant.

3. Utilisation des composés selon la revendication 1 comme tensioactifs dans l'industrie des peintures et vernis, en particulier comme agent antimoussant pour les dispersions aqueuses de polymères, éventuellement pigmentées.